# EUROPEAN PATENT SPECIFICATION

(11) **EP 3 730 605 B1**
(45) Date of publication and mention of the grant of the patent: **23.08.2023**
(21) Application number: 18892428.6
(22) Date of filing: 21.12.2018
(51) Int. Cl.: C12N 5/074

(54) **PLACENTA-DERIVED CELL-CONDITIONED MEDIUM FOR INDUCING DEDIFFERENTIATION FROM SOMATIC CELL TO INDUCED PLURIPOTENT STEM CELL AND DEDIFFERENTIATION-INDUCING METHOD USING SAME**
VON PLAZENTA STAMMENDES ZELLKONDITIONIERTES MEDIUM ZUR INDUZIERUNG DER ENTDIFFERENZIERUNG VON SOMATISCHEN ZELLEN VON INDUZIERTEN PLURIPOTENTEN STAMMZELLEN UND VERFAHREN ZUR INDUZIERUNG DER ENTDIFFERENZIERUNG
MILIEU CONDITIONNÉ PAR DES CELLULES DÉRIVÉES DU PLACENTA POUR L'INDUCTION D'UNE DÉDIFFÉRENCIATION À PARTIR DE CELLULES SOMATIQUES DONNANT DES CELLULES SOUCHES PLURIPOTENTES INDUITES ET PROCÉDÉ D'INDUCTION D'UNE DÉDIFFÉRENCIATION L'UTILISANT

(30) Priority: 22.12.2017 KR 20170178706
(43) Date of publication of application: 28.10.2020
(73) Proprietor: Korea University Research and Business Foundation, Seoul 02841 (KR)
(72) Inventor: KIM, Byoung Soo, Seoul 06005 (KR); LEE, Seung Jin, Seoul 03414 (KR); KANG, Ka Won, Seoul 03116 (KR)
(74) Representative: J A Kemp LLP
(86) International application number: PCT/KR2018/016519
(87) International publication number: WO 2019/125073

(56) References cited:
- WO-A1-2019/194549
- WO-A2-2012/008733
- WO-A2-2012/141471
- KR-A- 20090 076 989
- KR-A- 20120 118 407
- KR-B1- 101 395 214
- US-A1- 2012 264 215
- JI-HYE JUNG ET AL: "A Novel Culture Model for Human Pluripotent Stem Cell Propagation on Gelatin in Placenta-conditioned Media", JOURNAL OF VISUALIZED EXPERIMENTS, no. 102, 3 August 2015 (2015-08-03), XP055755848, DOI: 10.3791/53204
- JI-HYE JUNG ET AL: "CXCR2 and Its Related Ligands Play a Novel Role in Supporting the Pluripotency and Proliferation of Human Pluripotent Stem Cells", STEM CELLS AND DEVELOPMENT, vol. 24, no. 8, 15 April 2015 (2015-04-15) , pages 948-961, XP055755854, US ISSN: 1547-3287, DOI: 10.1089/scd.2014.0381
- JI-HYE JUNG ET AL: "CXCR2 Inhibition in Human Pluripotent Stem Cells Induces Predominant Differentiation to Mesoderm and Endoderm Through Repression of mTOR, [beta]-Catenin, and hTERT Activities", STEM CELLS AND DEVELOPMENT, vol. 25, no. 13, 1 July 2016 (2016-07-01), pages 1006-1019, XP055755853, US ISSN: 1547-3287, DOI: 10.1089/scd.2015.0395
- SEUNG-JIN LEE ET AL: "Generation of an induced pluripotent stem cell line KUMCi001-A from CD34+ bone marrow cells of a patient with acute lymphoblastic leukemia using human placenta-derived cell conditioned medium", STEM CELL RESEARCH, vol. 47, 1 August 2020 (2020-08-01), page 101913, XP055755839, NL ISSN: 1873-5061, DOI: 10.1016/j.scr.2020.101913
- SEUNG-JIN LEE ET AL: "Generation of an induced pluripotent stem cell line KUMCi001-A from CD34+ bone marrow cells of a patient with acute lymphoblastic leukemia using human placenta-derived cell conditioned medium", STEM CELL RESEARCH, vol. 47, 1 August 2020 (2020-08-01), page 101913, XP055755844, NL ISSN: 1873-5061, DOI: 10.1016/j.scr.2020.101913
- SEUNG-JIN LEE ET AL: "Generation of normal induced pluripotent stem cell line KUMCi002-A from bone marrow CD34+ cells of patient with multiple myeloma disease having 13q deletion and IGH translocation", STEM CELL RESEARCH, vol. 49, 1 December 2020 (2020-12-01), page 102030, XP055755846, NL ISSN: 1873-5061, DOI: 10.1016/j.scr.2020.102030
- OLIVEIRA, M. S. et al.: "Placental-derived Stem Cells: Culture, Differentiation and Challenges", World J. Stem Cells, vol. 7, no. 4, 26 May 2015 (2015-05-26), pages 769-775, XP055621231,
- EVANGELISTA, M. et al.: "Placenta-derived Stem Cells: New Hope for Cell Therapy?", Cytotechnology, vol. 58, no. 1, 28 September 2008 (2008-09-28), pages 33-42, XP019640998,

## Description

### [TECHNICAL FIELD]

The present invention relates to the use of a placenta-derived cell-conditioned medium for inducing dedifferentiation into induced pluripotent stem cells from somatic cells and a method for inducing dedifferentiation using the same.

### [BACKGROUND ART]

In order to produce stem cell therapeutic agents, large scale stem cell culture *in vitro* being a source thereof must be essentially carried out, and they must be safe and economical to be used as cell therapeutic agents in clinical practice.

However, for the proliferation culture of human induced pluripotent stem cells used at present, the method of using animal-derived support cells and the method of culturing in a container coated with a special gel containing animal-derived products may cause safety problems due to heterologous protein contamination. In case of using an expensive special gel, it is not suitable for mass production from economic perspectives.

When a placenta-derived cell-conditioned medium is employed, induced pluripotent stem cells can be cultured using animal-free and feederfree culture system, and proliferation and differentiation of pluripotent stem cells can be performed by the mechanism of CXCR2 which is a chemokine receptor.

Therefore, the present inventors anticipated that the placenta-derived cell-conditioned medium, which had been proven to be useful for culturing stem cells, can be utilized for the development of cell therapeutic agents by applying it to the dedifferentiation into induced pluripotent stem cells.

### [SUMMARY OF THE INVENTION]

The invention provides the use of a placenta-derived cell-conditioned medium (PCCM) for inducing dedifferentiation of somatic cells into induced pluripotent stem cells, wherein the placenta-derived cells of the PCCM are placenta-derived fibroblast-like cells isolated from the human chorionic plate.

The invention further provides a method for inducing dedifferentiation into induced pluripotent stem cells from somatic cells, comprising the following steps:
a somatic cell transformation step of transducing a nucleic acid sequence encoding at least one protein selected from the group consisting of OCT4, SOX2, c-Myc and KLF4 into somatic cells; and
a somatic cell culturing step of culturing the transformed somatic cells in a PCCM, wherein the placenta-derived cells of the PCCM are placenta-derived fibroblast-like cells isolated from the human chorionic plate.

### [DETAILED DESCRIPTION OF THE INVENTION]

The invention is defined by the independent claims. Further aspects and preferred embodiments are defined in the dependent claims. Any aspects, embodiments and examples of the present disclosure which do not fall under the scope of the appended claims do not form part of the invention and are merely provided for illustrative purposes.

### [Technical Problem]

The present inventors have made intensive researches to develop a placenta-derived cell-conditioned medium for inducing dedifferentiation into induced pluripotent stem cells (iPS) from somatic cells.

As a result, they have found that the dedifferentiation efficiency of induced pluripotent stem cells from somatic cells can be increased as compared with the case where a placenta-derived cell-conditioned medium is not used, thereby completing the present disclosure.

### [Technical Solution]

The present inventors have made intensive researches to develop the use of a placenta-derived cell-conditioned medium for inducing dedifferentiation into induced pluripotent stem cells from somatic cells. As a result, they have found that the dedifferentiation efficiency of induced pluripotent stem cells from somatic cells can be increased as compared with the case where a placenta-derived cell-conditioned media is not employed.

Hereinafter, embodiments of the present disclosure will be described in more detail.

One aspect of the present disclosure provides use of a placenta-derived cell-conditioned medium for inducing dedifferentiation into induced pluripotent stem cells (iPS) from somatic cells.

The somatic cell may be transformed with a nucleic acid sequence encoding at least one protein selected from the group consisting of OCT4, SOX2, c-Myc and KLF4, and for example, it may be transformed with a nucleic acid sequence encoding the OCT4 protein, a nucleic acid sequence encoding the SOX2 protein, a nucleic acid sequence encoding the c-Myc protein, and a nucleic acid sequence encoding the KLF4 protein.

The OCT4 protein may include an amino acid sequence of SEQ ID NO: 1, and for example, may be composed of the amino acid sequence of SEQ ID NO: 1, and may be interpreted to include sequences which have substantial identity thereto.

In addition, the nucleic acid encoding the OCT4 protein may encode the OCT4 protein including the amino acid sequence of SEQ ID NO: 1, and for example, may encode the OCT4 protein composed of the amino acid sequence of SEQ ID NO: 1, and may be interpreted to include sequences which have substantial identity thereto.

The SOX2 protein may include an amino acid sequence of SEQ ID NO: 2, and for example, may be composed of the amino acid sequence of SEQ ID NO: 2, and may be interpreted to include sequences which have substantial identity thereto.

Moreover, the nucleic acid encoding the SOX2 protein may encode the SOX2 protein including the amino acid sequence of SEQ ID NO: 2, and for example, may encode the SOX2 protein composed of the amino acid sequence of SEQ ID NO: 2, and may be interpreted to include sequences which have substantial identity thereto.

The c-Myc protein may include an amino acid sequence of SEQ ID NO: 3, and for example, may be composed of the amino acid sequence of SEQ ID NO: 3, and may be interpreted to include sequences which have substantial identity thereto.

Further, the nucleic acid encoding the c-Myc protein may encode the c-Myc protein including the amino acid sequence of SEQ ID NO: 3, and for example, may encode the c-Myc protein composed of the amino acid sequence of SEQ ID NO: 3, and may be interpreted to include sequences which have substantial identity thereto.

The KLF4 protein may include an amino acid sequence of SEQ ID NO: 4, and for example, may be composed of the amino acid sequence of SEQ ID NO: 4, and may be interpreted to include sequences interpreted to include sequences which have substantial identity thereto.

Further, the nucleic acid encoding the KLF4 protein may encode the KLF4 protein including the amino acid sequence of SEQ ID NO: 4, and for example, it may encode the KLF4 protein composed of the amino acid sequence of SEQ ID NO: 4, and may be interpreted to include sequences which have substantial identity thereto.

The substantial identity may be a sequence showing at least 60% homology, at least 70% homology, at least 80% homology, or at least 90% homology after fully aligning the target sequence with any other sequences and analyzing the aligned sequences using an algorithm commonly used in the art.

Alignment methods for comparison of sequences are known in the art, and for example, sequence analysis programs such as blastp, blastx, tblastn and tblastx can be used on the Internet using the Basic Local Alignment Search Tool (BLAST)of NCBI.

**[Table 1]**

| SEQ ID NO: | Na me | Sequence | Note |
|---|---|---|---|
| 1 | OC T4 | MAGHLASDFAFSPPPGGGGDGPGGPEPGWVDPRTWL SFQGPPGGPGIGPGVGPGSEVWGIPPCPPPYEFCGGMA YCGPQVGVGLVPQGGLETSQPEGEAGVGVESNSDGAS PEPCTVTPGAVKLEKEKLEQNPEESQDIKALQKELEQF AKLLKQKRITLGYTQADVGLTLGVLFGKVFSQTTICRF EALQLSFKNMCKLRPLLQKWVEEADNNENLQEICKAE TLVQARKRKRTSIENRVRGNLENLFLQCPKPTLQQISH IAQQLGLEKDVVRVWFCNRRQKGKRSSSDYAQREDF EAAGSPFSGGPVSFPLAPGPHFGTPGYGSPHFTALYSS VPFPEGEAFPPVSVTTLGSPMHSN | |
| 2 | SO X2 | | |
| 3 | c-My c | MDFFRVVENQQPPATMPLNVSFTNRNYDLDYDSVQP YFYCDEEENFYQQQQQSELQPPAPSEDIWKKFELLPTP PLSPSRRSGLCSPSYVAVTPFSLRGDNDGGGGSFSTAD | |
| | | QLEMVTELLGGDMVNQSFICDPDDETFIKNIIIQDCMW SGFSAAAKLVSEKLASYQAARKDSGSPNPARGHSVCS TSSLYLQDLSAAASECIDPSVVFPYPLNDSSSPKSCASQ DSSAFSPSSDSLLSSTESSPQGSPEPLVLHEETPPTTSSD SEEEQEDEEEIDVVSVEKRQAPGKRSESGSPSAGGHSK PPHSPLVLKRCHVSTHQHNYAAPPSTRKDYPAAKRVK LDSVRVLRQISNNRKCTSPRSSDTEENVKRRTHNVLER QRRNELKRSFFALRDQIPELENNEKAPKVVILKKATAY ILSVQAEEQKLISEEDLLRKRREQLKHKLEQLRNSCA | |
| 4 | KL F4 | MAVSDALLPSFSTFASGPAGREKTLRQAGAPNNRWRE ELSHMKRLPPVLPGRPYDLAAATVATDLESGGAGAAC GGSNLAPLPRRETEEFNDLLDLDFILSNSLTHPPESVAA TVSSSASASSSSSPSSSGPASAPSTCSFTYPIRAGNDPG VAPGGTGGGLLYGRESAPPPTAPFNLADINDVSPSGGF VAELLRPELDPVYIPPQQPQPPGGGLMGKFVLKASLSA PGSEYGSPSVISVSKGSPDGSHPVVVAPYNGGPPRTCP KIKQEAVSSCTHLGAGPPLSNGHRPAAHDFPLGRQLPS RTTPTLGLEEVLSSRDCHPALPLPPGFHPHPGPNYPSFL PDQMQPQVPPLHYQGQSRGFVARAGEPCVCWPHFGT HGMMLTPPSSPLELMPPGSCMPEEPKPKRGRRSWPRK RTATHTCDYAGCGKTYTKSSHLKAHLRTHTGEKPYH CDWDGCGWKFARSDELTRHYRKHTGHRPFQCQKCD RAFSRSDHLALHMKRHF | |

The somatic cell may be at least one selected from the group consisting of endothelial cells, epithelial cells and placental cells.

The placenta-derived cell is a placenta-derived fibroblast-like cell, which is isolated from the human chorionic plate and cultured.

The placenta-derived cell-conditioned medium may include human placenta-derived cells cultured in a cell growth medium.

Another aspect of the present disclosure but not part of the invention relates to a method for preparing a placenta-derived cell-conditioned medium for inducing dedifferentiation, including the following steps:
a placenta-derived cell culturing step of culturing human placenta-derived cells in a cell growth medium supplemented with a culture solution; and
a culture solution collecting step of collecting the culture solution including the human placenta-derived cell culture from the cell growth medium.

The placenta-derived cell may be a placenta-derived fibroblast-like cell, which is isolated from the human chorionic plate and cultured.

The culture solution of the culturing step may be Dulbecco's modified Eagle's medium (DMEM)/F-12, and for example, it may be DMEM containing 10% FBS, 10% penicillin and 10% sodium pyruvate, or a highglucose medium.

The culture solution may further include a serum replacement agent, and may be, for example, KnockOut^{™} Serum Replacement (KnockOut^{™} SR), but is not limited thereto.

The culture solution collected in the collection step may include a placenta-derived cell culture, for example, a human placenta-derived cell culture.

Yet another aspect of the present disclosure relates to a method for inducing dedifferentiation into induced pluripotent stem cells (iPS) from somatic cells, including the following steps:
a somatic cell transformation step of transducing a nucleic acid sequence encoding at least one protein selected from the group consisting of OCT4, SOX2, c-Myc and KLF4 into somatic cells; and
a somatic cell culturing step of culturing the transformed somatic cells in a placenta-derived cell-conditioned medium.

The somatic cell may be at least one selected from the group consisting of endothelial cells, epithelial cells and placental cells.

The placenta-derived cell is a placenta-derived fibroblast-like cell, which is isolated from the human chorionic plate and cultured.

The method for inducing dedifferentiation may further include a stem cell isolation step of isolating stem cells from colonies formed during the somatic cell culturing step.

The stem cell isolation step may be performed by staining with a stem cell marker, and for example, it may be performed by staining with Tra-60, alkaline phosphatase, SSEA4, TRA-1-60 and TRA-1-80, and may be isolated through a live stain in order to confirm whether the formed colonies are stem cells.

The method for inducing dedifferentiation may further include a stem cell activity verification step of confirming the activity of at least one protein selected from the group consisting of OCT-4, NANOG, SSEA-4 and Tra-81 in the stem cells isolated from colonies.

The activity verification step may be performed by comparing the reprogramming efficiency through an alkaline phosphatase staining after transducing a dedifferentiation factor for 7 days and inducing dedifferentiation stem cells for 3 days in the placenta-derived cell-conditioned medium or E8, but is not limited thereto.

The method for inducing dedifferentiation may further include a stem cell differentiation ability verification step of confirming the differentiation ability into ectoderm, mesoderm or endoderm by forming embryonic cells *in vitro* using the stem cells isolated from colonies.

The verification of the differentiation ability into the ectoderm may be performed with at least one antibody selected from the group consisting of TUJ1, Nestin, Otx2, SOX1 and Pax6, but is not limited thereto.

The verification of the differentiation ability into the mesoderm may be performed with at least one antibody selected from the group consisting of Desmin, Brachyury, HAND1 and Snail, but is not limited thereto.

The verification of the differentiation ability into the endoderm may be performed with at least one antibody selected from the group consisting of AFP, GATA-4, SOX17, HNF4A and FOXA2, but is not limited thereto.

### [ADVANTAGEOUS EFFECTS]

The present disclosure relates to the use of a placenta-derived cell-conditioned medium for inducing dedifferentiation into induced pluripotent stem cells from somatic cells and a method for inducing dedifferentiation using the same. When the placenta-derived cell-conditioned medium for inducing dedifferentiation according to the present disclosure is employed, personalized dedifferentiation stem cells can be stably established using a medium composed of human-derived products only. In addition, the dedifferentiation efficiency of induced pluripotent stem cells from somatic cells can be increased as compared with the case where a placenta-derived cell-conditioned medium is not used.

### [BRIEF DESCRIPTION OF DRAWINGS]

FIG. 1 is a schematic diagram showing a process for inducing dedifferentiation into induced pluripotent stem cells from somatic cells.
FIG. 2 shows images showing morphological changes in the process of dedifferentiation into induced pluripotent stem cells from somatic cells.
FIG. 3a is an image showing the results of staining ALP, a stem cell marker for the induced pluripotent stem cells prepared by inducing dedifferentiation.
FIG. 3b is a graph showing the results of comparing the dedifferentiation efficiency of induced pluripotent stem cells in the placenta-derived cell-conditioned medium relative to normal medium.
FIG. 4a shows images showing the results of confirming the expression of the stem cell-specific gene through an immunohistochemical method for the induced pluripotent stem cells prepared by inducing dedifferentiation.
FIG. 4b is a graph showing the results of confirming the expression of the stem cell-specific gene through a polymerase chain reaction for the induced pluripotent stem cells prepared by inducing dedifferentiation.
FIG. 5a is an image showing the results of confirming the karyotype through chromosomal analysis for the induced pluripotent stem cells prepared by inducing dedifferentiation in vascular endothelial cells.
FIG. 5b is an image showing the results of confirming the karyotype through chromosomal analysis for the induced pluripotent stem cells prepared by inducing dedifferentiation in placental cells.
FIG. 5c is an image showing the results of confirming the karyotype through chromosomal analysis for the induced pluripotent stem cells prepared by inducing dedifferentiation in fibroblasts .
FIG. 6a is an image showing the results of confirming the differentiation ability into ectoderm, mesoderm and endoderm by forming teratomas *in vitro* with the induced pluripotent stem cells prepared by inducing dedifferentiation from somatic cells .
FIG. 6b is an image showing the results of verifying the formation of teratomas in immune-deficient mice with the induced pluripotent stem cells prepared by inducing dedifferentiation from somatic cells.
FIG. 6c is an image showing the results of verifying the formation of teratomas in immune-deficient mice with the induced pluripotent stem cells prepared by inducing dedifferentiation from somatic cells.

Use of placenta-derived cell-conditioned medium for inducing dedifferentiation into induced pluripotent stem cells from somatic cells and method for inducing dedifferentiation using the same.

### [DETAILED DESCRIPTION]

A placenta-derived cell-conditioned medium for inducing dedifferentiation into induced pluripotent stem cells (iPS) from somatic cells.

Hereinafter, the present disclosure will be described in more detail by way of Examples. However, these Examples are merely provided to more specifically describe the present disclosure, and it is obvious to those skilled in the art that, according to the gist of the present disclosure, the scope of the present disclosure is not limited to or by these examples.

### Example 1: Dedifferentiation into Induced Pluripotent Stem Cells from Somatic Cells

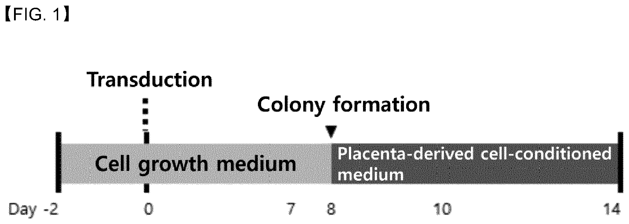

As shown in FIG. 1, endothelial cells (Primary Umbilical Vein Endothelial Cells, ATCC #PCS-100-010), epidermal Cells (Primary Dermal Fibroblasts, ATCC # PCS-201-012) and placental cells, which are three types of human somatic cells, were transformed with Sandy virus dedifferentiation factors (OCT4, SOX2, c-Myc and KLF4) and incubated in the growth medium provided for 7 days.

The placental cells were obtained from placental tissues isolated by surgical operation through a cesarean section after a written consent from a healthy pregnant woman who received therapeutic abortion at 7 weeks of pregnancy.

In detail, cells were isolated from chorionic tissues of the placenta, and the isolated placental cells were incubated in Dulbecco's modified Eagle's medium (DMEM) containing 20% fetal bovine serum (FBS), 100 U/ml penicillin and 100 g/ml streptomycin in a flask coated with 0.1 % gelatin for one week.

Transformation was performed by purchasing a kit (CytoTune^{™}-iPS 2.0 Sendai Reprogramming Kit, Life Technologies).

The transformed somatic cells were transplanted into a new culture vessel, and after 8 days, the cells were incubated in the placenta-derived cell-conditioned medium provided, and colonies were formed within 24 hours.

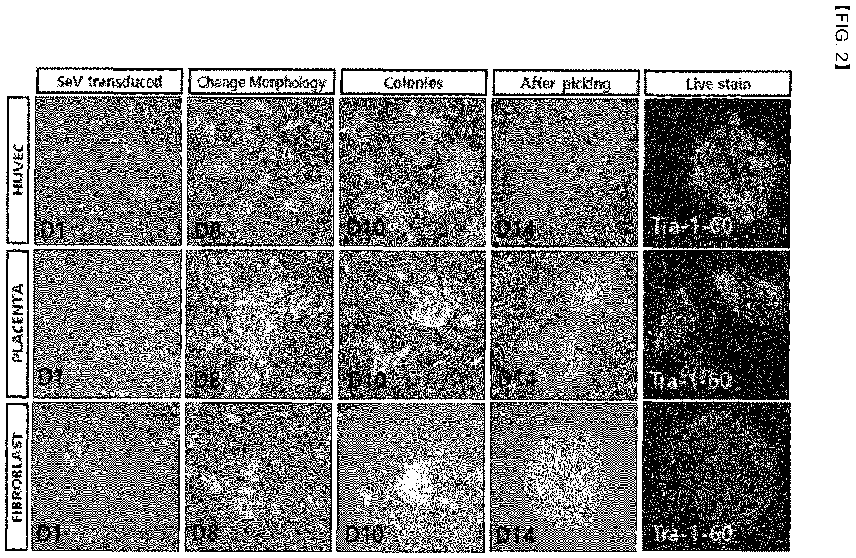

As shown in FIG. 2, among these, the dedifferentiation stem cells, which were verified by staining with Tra-60 which is a stem cell marker, were selectively isolated and cultured. At this time, a commercialized culture solution (define Essential 8 medium: E8) was used as a control.

### Example 2: Confirmation of Dedifferentiation Stem Cells

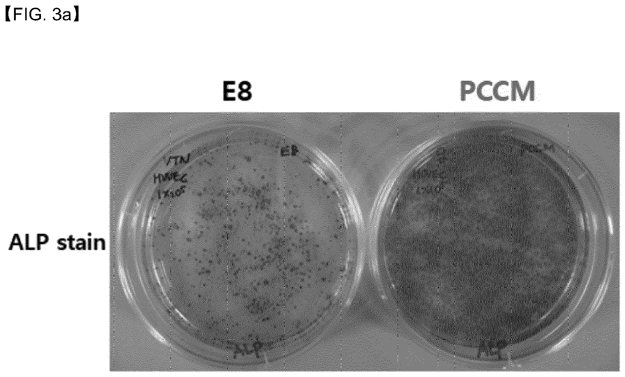

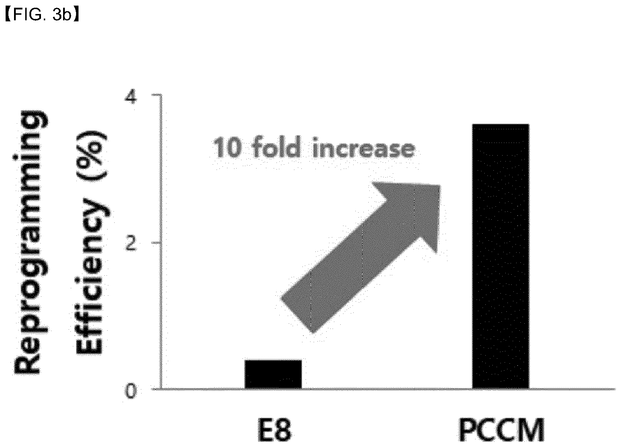

It was confirmed through an alkaline phosphatase staining whether the dedifferentiation stem cells induced from the somatic cells exhibited a self-renewal ability, which are characteristics of induced pluripotent stem cells, and the efficiency thereof was compared with a control group in which the dedifferentiation was induced in E8 medium. The alkaline phosphatase staining was performed using a kit (ES Cell Characterization kit, Chemicon International), and the results are shown in FIG. 3a. These were calculated as efficiency (%) and plotted as a graph, the results of which are shown in FIG. 3b and Table 2.

**[Table 2]**

| Condition | Cells plated | Colonies/well | Efficiency (%) |
|---|---|---|---|
| E8 | 1 X 10⁵ | 312 ±0.05 | 0.312 |
| PCCM | 1 X 10⁵ | 3921 ±0.01 | 3.921 |

As can be seen in FIG. 3a, it could also be observed with the naked eye that there were a significant number of cells showing the characteristics of the induced pluripotent stem cells in the placenta-derived cell-conditioned medium. In addition, as can be confirmed in FIG. 3b and Table 2, in a total of 100,000 cells, 312±0.05 cells showed ALP activity in E8, and 3921±0.01 cells in PCCM, and the dedifferentiation efficiency in the placenta-derived cell-conditioned medium was found to be about 10 times higher than in the control group.

### Example 3: Confirmation of Specificity of Dedifferentiation Stem Cells

### 3-1. Confirmation of Specific Marker Expression Level

The function of stem cells was verified by confirming the expression levels of OCT-4, NANOG, SSEA-4, and Tra-81, which are specific markers for induced pluripotent stem cells. In order to confirm whether the three established stem cell lines retained their properties, the expression of markers specific for dedifferentiation stem cells was confirmed using an immunofluorescence staining. First, cells were cultured on a cover slip for the immunofluorescence staining, and the expression of the stem cell-specific markers OCT-4 and SSEA-4 was measured by immunofluorescence staining.

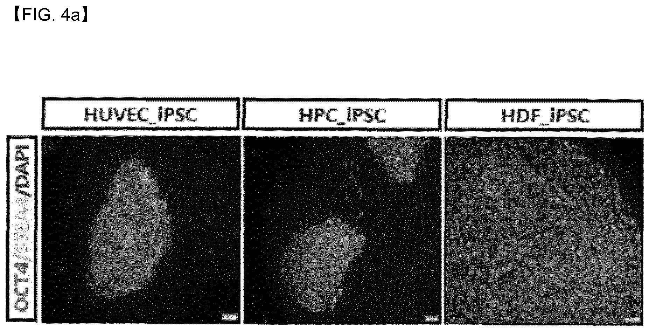

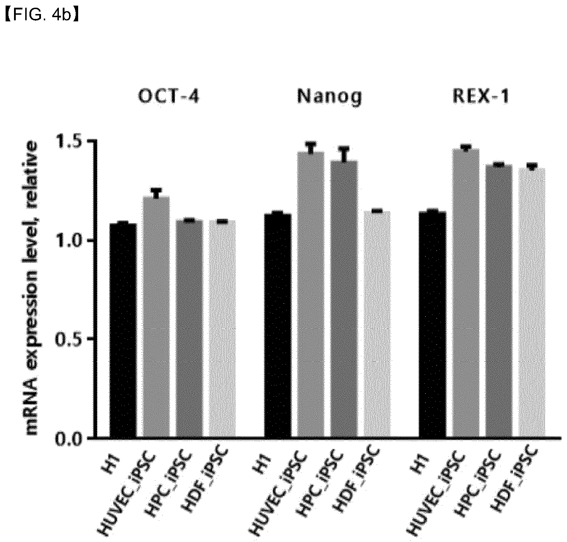

Specifically, when the cells were grown to 70 to 80%, they were fixed with 4% paraformaldehyde for 10 minutes. Then, 0.1% Triton X100 was infiltrated into the cells for 10 minutes, and the primary antibody OCT-4 (Cell Signaling Technology #2750) and SSEA-4 (Millipore # MAB4304) were diluted at 1:1000 and treated to the cells. Then, the cells were incubated overnight at 4°C. The next day, the cells were treated with the secondary antibody at room temperature for 1 hour and with 4',6-diamidino-2-phenylindole (DAPI), allowed to stand for 5 minutes under dark conditions, and then observed under a fluorescence microscope. Then, the mRNA expression levels of OCT-4, Nanog, and REX-1, the neural stem cell-specific factors, were measured by a real-time PCR (Quantitative real-time PCR Analysis). Specifically, RNA was isolated from the cells induced by dedifferentiation stem cells using a kit (Qiagen RNeasy kit, Qiagen Hilden, Germany), and cNDA was synthesized using 2ug of RNA, oligo(dT) and reverse transcriptase (Superscript II reverse transcriptase, Gibco). Primer of the OCT-4, Nanog and REX-1 genes shown in Table 3 below and master mix (iQ SYBR Green qPCR Master Mix) were added to each of the synthesized cDNAs and analyzed using a device (Bio-Rad iCycler iQ system, Bio-Rad Laboratories, USA), and the results are shown in FIGS. 4a and 4b, and Table 4.

**[Table 3]**

| SEQ ID NO: | Name | Sequence (5->3) | Note |
|---|---|---|---|
| 5 | OCT-4_F | TCTCGCCCCCTCCAGGT | |
| 6 | OCT-4_R | CTGCTTCGCCCTCAGGC | |
| 7 | Nanog_F | AAAGAATCTTCACCTATGCC | |
| 8 | Nanog_R | GAAGGAAGAGGAGAGACAGT | |
| 9 | REX-1_F | CAGATCCTAAACAGCTCGCAGAAT | |
| 10 | REX-1_R | GCGTACGCAAATTAAAGTCCAGA | |
| 11 | GAPDH_F | GAGTCCACTGGCGTCTTCAC | |
| 12 | GAPDH_R | TTCACACCCATGACGAACAT | |

**[Table 4]**

| | H1 control | HUVEC_iPSC | PLACENTA_iPSC | FIBROBLAST_iPSC |
|---|---|---|---|---|
| OCT-4 | 1.0733333 | 1.21 | 1.09 | 1.0923333 |
| Nanog | 1.1233333 | 1.4333333 | 1.39 | 1.1366667 |
| REX-1 | 1.1333333 | 1.4466667 | 1.3666667 | 1.35 |

As can be confirmed in FIG. 4a, the stem cell-specific markers OCT4 and SSEA4 were strongly expressed in the dedifferentiation stem cells. In addition, as can be confirmed in FIG. 4b and Table 4, the content of the intracellular induced pluripotent stem cell specific-protein in the dedifferentiation stem cells was found to be higher than in the human embryonic stem cell line H1 (WiCell, Wisconsin, USA) as a control.

### 3-2. Chromosome Analysis

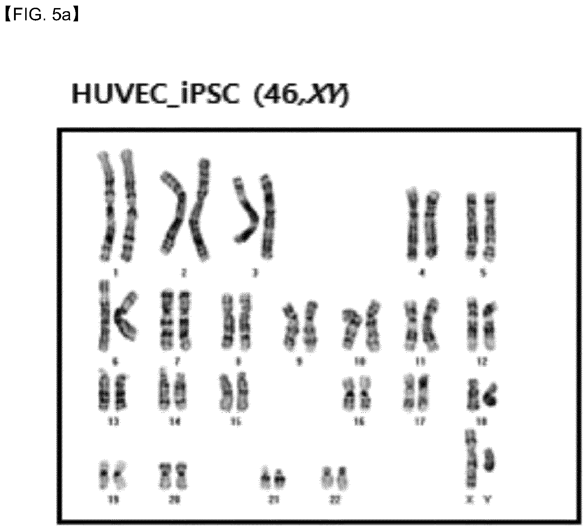

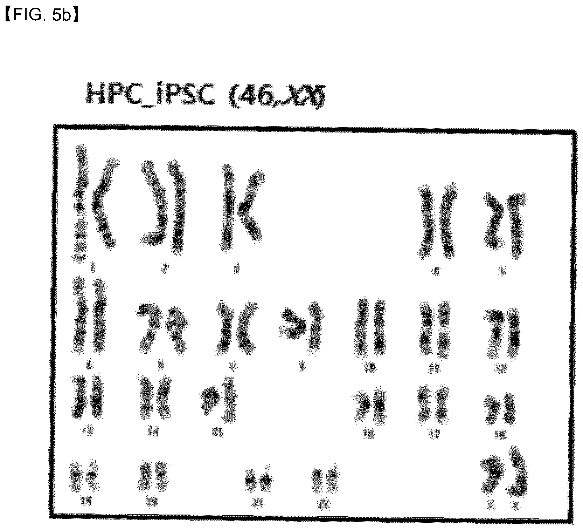

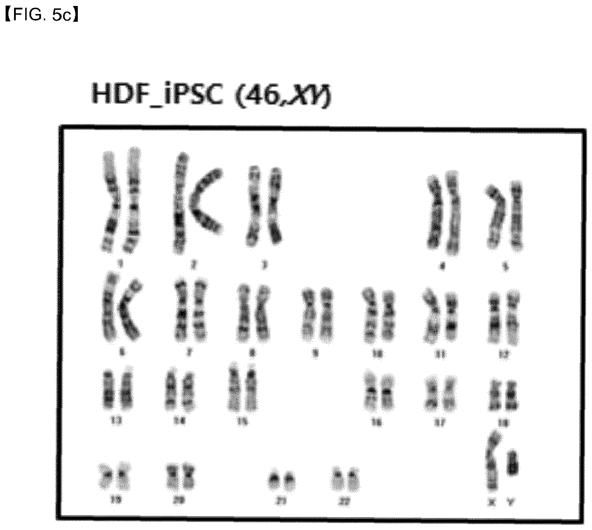

In order to confirm whether the dedifferentiation stem cells maintain normal karyotypes, chromosome analysis was performed to verify the stability. In detail, for the chromosome analysis, 0.1 g/ml of colcemid was treated to the dedifferentiation stem cells at 1.5 × 10⁶ cells for 3 to 4 hours. Then, 0.25% trypsin-EDTA was treated for 5 minutes to isolate the cells from the culture dish, and then, the cells were incubated in a 0.075M KC1 solution at 37°C for 20 minutes. Then, methanol and acetic acid were mixed at a ratio of 3:1 to fix the cells, and the karyotypes of the established dedifferentiation stem cells were measured at a resolution of 300 band level, and the results are shown in FIGS. 5a to 5c.

As can be shown in FIGS. 5a to 5c, it was confirmed through chromosome analysis that the dedifferentiation stem cells maintained normal karyotypes. The characteristics and stability were verified through the specific markers in the dedifferentiation stem cells established with high efficiency from a total of three various somatic cells using the placenta-derived conditioned medium.

### Example 4: Confirmation of Differentiation ability of Dedifferentiation Stem Cells

### 4-1. Confirmation of Differentiation ability

The differentiation ability was confirmed by forming embryonic cells *in vitro* to determine whether the dedifferentiated induced pluripotent stem cells have the ability to differentiate into ectoderm, mesoderm, and endoderm. In detail, in order to confirm the differentiation ability *in vitro,* embryonic cells were formed for 2 weeks in a non-adherent culture vessel, and immunofluorescence was performed to confirm whether the cells could each differentiate into ectoderm, mesoderm and endoderm.

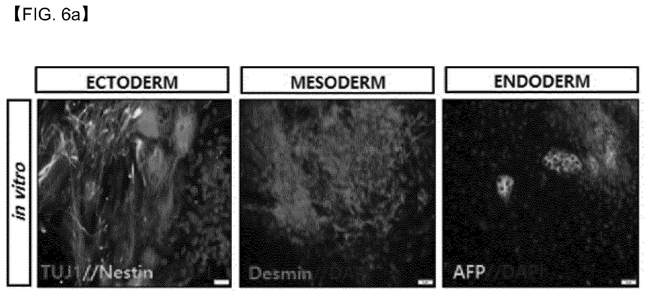

Specifically, after fixing the cells with 4% paraformaldehyde for 10 minutes, 0.1% Triton X100 was infiltrated into the cells for 15 minutes and then blocked with PBS containing 3% horse serum for 1 hour. Then, the primary antibody TUJ1 (COVANCE #MRB-435P), Nestin (Abcam #ab22035), Desmin (Santacruz #sc-14026), and AFP (Santacruz #sc-166335) were diluted at 1:1000 and treated to the cells, and the cells were incubated overnight at 4°C. The next day, the cells were treated with the secondary antibody, incubated at room temperature for 1 hour, to which 4',6-diamidino-2-phenylindole (DAPI) was added, allowed to stand for 5 minutes in dark conditions, and then observed under a fluorescence microscope. The results are shown in FIG. 6a.

As can be shown in FIG. 6a, the neuroepithelium differentiated into ectoderm was identified using Tuj1 and Nestin markers, and the cartilage differentiated into mesoderm was identified using Desmin as mesodermal marker. In addition, the intestinal epithelium differentiated into endoderm was verified using immunohistochemistry. It was verified by fluorescence immunoassay that differentiation into ectoderm was made from the formation of embryonic cells *in vitro,* and Desmin as a mesodermal marker and AFP as an endoderm marker were expressed.

### 4-2. Verification of Teratoma Formation

In order to confirm the differentiation ability *in vivo,* it was verified whether teratomas were formed in immune-deficient mice. In detail, the dedifferentiated induced pluripotent stem cells were injected into the subcutaneous tissue of immune-deficient mice at 1.0 X 10⁶ cells. After 12 weeks, the formation of teratoma was verified using immunohistochemistry.

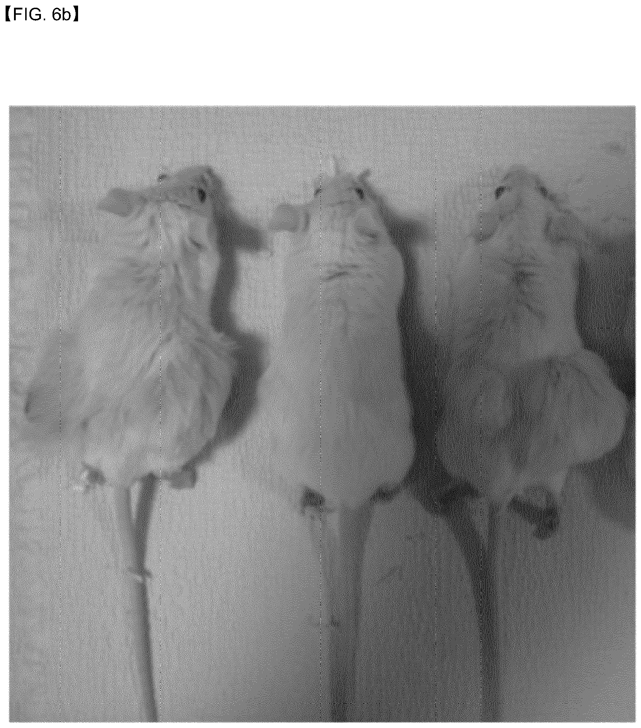

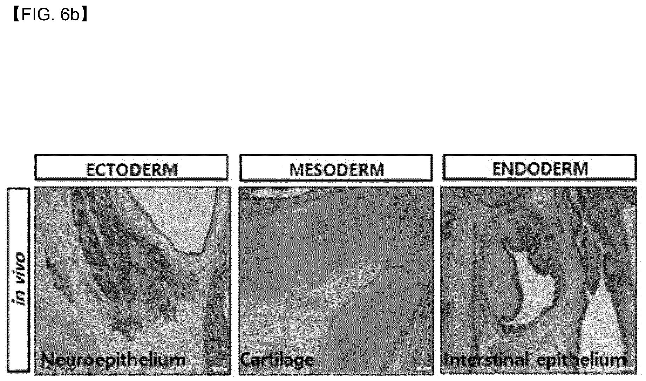

In detail, euthanasia was performed using carbon dioxide gas when the formed teratoma was ≥1 cm³ in size. The teratoma was removed through surgical procedures and fixed in 4% formaldehyde. After dehydration, the tissue was immersed in xylene for a long time to clean the tissue. The tissue was placed in a liquid paraffin container and immersed therein in an oven at 60°C. The tissue was embedded in a mold, attached to a slide glass, dried, then placed in an oven at 60°C and subjected to deparaffinization for about a day. The eosin (E), in which Harris hematoxylin (H) was exposed at room temperature for 30 seconds, was incubated at room temperature for 1 minute and subjected to histological analysis, and the results are shown in FIGS. 6b and 6c.

As can be confirmed in FIG. 6b and 6c, it was observed that teratoma was formed in the subcutaneous tissue of the immune-deficient mice. As a result, by confirming the differentiation ability of dedifferentiated induced pluripotent stem cells *in vivo* and *in vitro,* the ability of the dedifferentiated stem cells to differentiate into desired cells using the human placenta-derived conditioned medium was verified.

### [Industrial Applicability]

The present disclosure provides a placenta-derived cell-conditioned medium for inducing dedifferentiation into induced pluripotent stem cells from somatic cells and a method for inducing dedifferentiation using the same.

## Claims

1. Use of a placenta-derived cell-conditioned medium (PCCM) for inducing dedifferentiation of somatic cells into induced pluripotent stem cells, wherein the placenta-derived cells of the PCCM are placenta-derived fibroblast-like cells isolated from the human chorionic plate.

2. The use of claim 1, wherein the somatic cell is at least one selected from the group consisting of endothelial cells, epithelial cells and placental cells.

3. A method for inducing dedifferentiation into induced pluripotent stem cells from somatic cells, comprising the following steps:
a somatic cell transformation step of transducing a nucleic acid sequence encoding at least one protein selected from the group consisting of OCT4, SOX2, c-Myc and KLF4 into somatic cells; and
a somatic cell culturing step of culturing the transformed somatic cells in a PCCM, wherein the placenta-derived cells of the PCCM are placenta-derived fibroblast-like cells isolated from the human chorionic plate.

4. The method of claim 3, wherein the somatic cell is at least one selected from the group consisting of endothelial cells, epithelial cells and placental cells.

5. The method of claim 3, wherein the method for inducing dedifferentiation further comprises a stem cell isolation step of isolating stem cells from colonies formed during the somatic cell culturing step.

6. The method of claim 5, wherein the stem cell isolation step is performed by staining with a stem cell label marker.

7. The method of claim 5, wherein the method for inducing dedifferentiation further comprises a stem cell activity verification step of confirming the activity of at least one protein selected from the group consisting of OCT-4, NANOG, SSEA-4 and Tra-81 in the stem cells isolated from colonies.

8. The method of claim 5, wherein the method for inducing dedifferentiation further comprises a stem cell differentiation ability verification step of confirming the differentiation ability into ectoderm, mesoderm or endoderm by forming embryonic cells *in vitro* using the stem cells isolated from colonies.

## Patentansprüche

1. Verwendung eines von der Plazenta stammenden zellkonditionierten Mediums (Placenta-derived Cell-Conditioned Medium, PCCM) zur Induktion der Entdifferenzierung von somatischen Zellen in induzierte pluripotente Stammzellen, wobei die von der Plazenta stammenden Zellen des PCCM von der Plazenta stammende fibroblastenähnliche Zellen sind, die aus der menschlichen Chorionplatte isoliert wurden.

2. Verwendung nach Anspruch 1, wobei die somatische Zelle mindestens eine Zelle ist, die aus der Gruppe ausgewählt ist, die aus Endothelzellen, Epithelzellen und Plazentazellen besteht.

3. Verfahren zur Induktion der Entdifferenzierung in induzierte pluripotente Stammzellen aus somatischen Zellen, das folgende Schritte umfasst:
einen somatischen Zelltransformationsschritt der Transduktion einer Nukleinsäuresequenz, die für mindestens ein Protein kodiert, das aus der Gruppe ausgewählt ist, die aus OCT4, SOX2, c-Myc und KLF4 besteht, in somatische Zellen; und
einen Schritt der Kultivierung somatischer Zellen, bei dem die transformierten somatischen Zellen in einem PCCM kultiviert werden, wobei die von der Plazenta stammenden Zellen der PCCM von der Plazenta stammende fibroblastenähnliche Zellen sind, die aus der menschlichen Chorionplatte isoliert wurden.

4. Verfahren nach Anspruch 3, wobei die somatische Zelle mindestens eine Zelle ist, die aus der Gruppe ausgewählt ist, die aus Endothelzellen, Epithelzellen und Plazentazellen besteht.

5. Verfahren nach Anspruch 3, wobei das Verfahren zur Induktion der Entdifferenzierung ferner einen Schritt der Stammzellenisolierung umfasst, bei dem Stammzellen aus den während des Kultivierungsschritts der somatischen Zellen gebildeten Kolonien isoliert werden.

6. Verfahren nach Anspruch 5, wobei der Schritt der Stammzellisolierung durch Färben mit einem Stammzellmarker durchgeführt wird.

7. Verfahren nach Anspruch 5, wobei das Verfahren zur Induktion der Entdifferenzierung ferner einen Schritt zur Verifizierung der Stammzellaktivität umfasst, bei dem die Aktivität mindestens eines Proteins, das aus der Gruppe ausgewählt ist, die aus OCT-4, NANOG, SSEA-4 und Tra-81 besteht, in den aus Kolonien isolierten Stammzellen bestätigt wird.

8. Verfahren nach Anspruch 5, wobei das Verfahren zur Induktion der Entdifferenzierung ferner einen Schritt zur Verifizierung der Differenzierungsfähigkeit der Stammzellen umfasst, bei dem die Differenzierungsfähigkeit in Ektoderm, Mesoderm oder Endoderm durch Bildung embryonaler Zellen in vitro unter Verwendung der aus Kolonien isolierten Stammzellen bestätigt wird.

## Revendications

1. Utilisation d'un milieu conditionné par cellules dérivées de placenta (PCCM) pour provoquer une dédifférenciation de cellules somatiques en cellules souches pluripotentes provoquées, dans laquelle les cellules dérivées de placenta du PCCM sont cellules de type fibroblaste dérivées de placenta isolées de plaque choriale humaine.

2. Utilisation selon la revendication 1, dans laquelle la cellule somatique est au moins une sélectionnée parmi le groupe constitué de : cellules endothéliales, cellules épithéliales et cellules placentaires.

3. Procédé pour provoquer une dédifférenciation en cellules souches pluripotentes provoquées, à partir de cellules somatiques, comprenant les étapes suivantes :
une étape de transformation de cellule somatique, consistant à effectuer une transduction d'une séquence d'acide nucléique codant au moins une protéine sélectionnée parmi le groupe constitué de : OCT4, SOX2, c-Myc et KLF4, en cellules somatiques ; et
une étape de culture de cellule somatique, consistant effectuer la culture des cellules somatiques transformées, dans un PCCM, dans lequel les cellules dérivées de placenta du PCCM sont des cellules de type fibroblaste dérivées de placenta, isolées de plaque choriale humaine.

4. Procédé selon la revendication 3, dans lequel la cellule somatique est au moins une sélectionnée parmi le groupe constitué de : cellules endothéliales, cellules épithéliales et cellules placentaires.

5. Procédé selon la revendication 3, dans lequel le procédé pour provoquer une dédifférenciation comprend en outre une étape d'isolation de cellules souches, constituant à isoler des cellules souches, de colonies formées durant l'étape de culture de cellules somatiques.

6. Procédé selon la revendication 5, dans lequel l'étape d'isolation de cellules souches est réalisée par coloration avec un marqueur de cellules souches.

7. Procédé selon la revendication 5, dans lequel le procédé pour provoquer une dédifférenciation comprend en outre une étape de vérification d'activité de cellules souches, consistant à confirmer l'activité d'au moins une protéine sélectionnée parmi le groupe constitué de : OCT-4, de NANOG, de SSEA-4 et de Tra-81, dans les cellules souches isolées de colonies.

8. Procédé selon la revendication 5, dans lequel le procédé pour provoquer une dédifférenciation comprend en outre une étape de vérification de capacité de différenciation de cellules souches, consistant à confirmer la capacité de différenciation en ectoderme, mésoderme ou endoderme, en formant des cellules embryonnaires *in vitro* en utilisant les cellules souches isolées de colonies.
